# EUROPEAN PATENT APPLICATION

(11) **EP 2 281 561 A2**
(43) Date of publication of application: **09.02.2011**
(21) Application number: 10184940.4
(22) Date of filing: 11.08.2003
(51) Int. Cl.: A61K 31/404, A61K 31/454, A61K 31/517, A61K 45/06, A61P 37/00, A61P 37/06, A61P 29/00, A61P 1/00, A61P 3/10, A61P 17/06, A61P 11/06, A61P 25/00

(54) **Use of LCK inhibitors for treatment of immunologic diseases**

(30) Priority: 16.08.2002 DE 10237423
(62) Divisional of application: 03792292.9
(71) Applicant: Boehringer Ingelheim Pharma GmbH & Co. KG, 55216 Ingelheim am Rhein (DE)
(72) Inventor: Roth, Gerald Jürgen, 88400 Biberach (DE); Hauptmann, Rudolf, 2483 Ebreichsdorf (AT); Hilberg, Frank, 1050 Wien (AT); Colbatzky, Florian, 88441 Stafflangen (DE); Ernst, Steffen, 43537 Mölnlycke (DE); Heckel, Armin, 88400 Biberach (DE); Stefanic, Martin, Friedrich, 88447 Warthausen (DE); Walter, Rainer, 88400 Biberach (DE)
(74) Representative: Hammann, Heinz

(57) **Abstract**

The invention relates to a method of treating immunologic diseases or pathological conditions involving an immunologic component using certain Lck inhibitors already known as kinase inhibitors for therapy in oncology, optionally in combination with one or more other drugs selected from NSAIDs, steroids, DMARDs, immunsuppressives, biologic response modifiers and antinfectives, pharmaceutical compositions comprising said Lck inhibitors together with said other drugs, and the use of the Lck inhibitors for the manufacture of a pharmaceutical composition for the treatment of immunologic diseases or pathological conditions involving an immunologic component.

## Description

### TECHNICAL FIELD OF THE INVENTION

This invention relates to a method of treating immunologic diseases or pathological conditions involving an immunologic component using a compound selected from compounds (A) to (AL) listed below already known as kinase inhibitors for therapy in oncology, and the use of these compounds for the manufacture of a pharmaceutical composition for the treatment of said immunologic diseases or pathological conditions.

### BACKGROUND OF THE INVENTION

Compounds (A) to (AL) listed below, their preparation as well as the pharmacological activity of these compounds based on inhibition of kinases, e.g. VEGFR-2, suitable for therapy in oncology, are disclosed in WO 02/36564, WO 99/52869, WO 00/18734, WO 00/73297, WO 01/27080, WO 01/27081 and WO 01/32651. The cited documents are herewith incorporated by reference with respect to any aspects disclosed relating to these specific compounds.

Lck, a further tyrosine kinase belonging to the src family of tyrosine kinases not mentioned in the references cited above, is functionally required for T-cell activation through the T-cell antigen receptor (TCR) (see A.E. Nel: T-cel) activation through antigen receptor. Part 1: Signaling components, signaling pathways, and signal integration at the T-cell antigen receptor synapse. J. Allergy Clin Immunol, 109, 5, 758-770, 2002) and possibly T-cell survival (Seddon, B.; Legname, G.; Tomlinson, P.; Zamoyska, R. : Long-term survival but impaired homeostatic proliferation of naive T cells in the absence of p56(lck). Science 290: 127-131, 2000). Therefore a Lck inhibitor has a high possible therapeutic potential in the treatment of T-cell mediated diseases, e.g. in the treatment of immunologic diseases. Certain autoimmune diseases such as inflammatory diseases (for example inflammatory bowel disease, rheumatoid arthritis, glomerulonephritis and lung fibrosis, psoriasis, hypersensitivity reactions of the skin, atherosclerosis, restenosis, allergic asthma, multiple sclerosis and type 1 diabetes are believed to be associated with inappropriate T cell activation (J. H. Hanke et al., Inflamm. Res., 1995, 357). In addition the acute rejection of transplanted organs as well as Graft versus Host Disease (GvHD) after allogeneic bone marrow and stem cell transplantation can also be interpreted as consequence of inappropriate T cell activation. Lck inhibitors offer an approach for treatment of the indications mentioned hereinbefore. Agents of this kind would offer therapy for transplant rejection and autoimmune diseases whilst avoiding toxicities associated with the commonly used, less selective immunosuppressants. The leading agent for prevention or treatment of transplant rejection is cyclosporin A which, although effective, is often associated with side-effects such as renal damage and hypertension which results in kidney failure in a substantial number of patients. It is contemporary practice to treat rheumatoid arthritis initially with symptom relief agents such as NSAIDs, which have no effect on disease progression and are often associated with unwanted side-effects. A rationally based, disease modifying agent, without such deleterious side-effects, would therefore offer significant benefits in the prevention or treatment of transplant rejection or autoimmune conditions such as rheumatoid arthritis.

There is considerable evidence that VEGF plays a key role in the pathogegenesis in rheumatoid arthritis, especially in the formation of the pannus (Paleolog EM, Arthritis Res 2002;4 Suppl 3:S81-90, Pavonen et al, J Rheumatol 2002 Jan;29(1):39-45, Afuwape AO et al, Histol Histopathol 2002;17(3):961-72). Thus, combined inhibition of VEGFR-tyrosine kinases and Lck is considered of potentially high benefit for patients with this disease. The same considerations can be applied to psoriasis and inflammatory bowel disease (Folkman J, Nat Med. 1995 Jan;1(1):27-31. Review; Griga T et al, Hepatogastroenterology 2002 Jan-Feb;49(43):116-23, Creamer D et al, Arch Dermatol 2002 Jun;138(6):791-6).

### BRIEF SUMMARY OF THE INVENTION

In view of the work cited above there is a clear need for compounds being active as Lck inhibitors in order to treat T-cell mediated diseases, e.g. in the treatment of immunologic diseases or pathological conditions involving an immunologic component.

It is therefore an object of the invention to provide a method for treating immunologic diseases or pathological conditions involving an immunologic component comprising administering to a patient in need of such treatment an effective amount of a pharmaceutical composition comprising a compound selected from compounds (A) to (AL) already known as agents for therapy in oncology.

A second object of the invention is a pharmaceutical composition comprising a. - compound selected from compounds (A) to (AL) together with one or more other drugs selected from nonsteroidal anti-inflammatory drugs (NSAIDs), steroids, disease-modifying antirheumatic drugs (DMARDs), immunsuppressives, biologic response modifiers and antinfectives for use in treatment of immunologic diseases or pathological conditions involving an immunologic component.

A third object of the invention is the use of a compound selected from compounds (A) to (AL) for the manufacture of a pharmaceutical composition for the treatment of immunologic diseases or pathological conditions involving an immunologic component.

### DETAILED DESCRIPTION OF THE INVENTION

It has now surprisingly been found that compounds
- (A): (Z)-3-(1-(4-(piperidin-1-yl-methyl)-phenylamino)-1-phenyl-methylene)-5- (methylsulfonylamino)-2-indolinone;

- (B): (Z)-3-(1-(4-(piperidin-1-yl-methyl)-phenylamino)-1-phenyl-methylene)-5-(ethyl- sulfonylamino)-2-indolinone;
- (C): (Z)-3-(1-(4-(dimethylaminomethyl)-phenylamino)-1-phenyl-methylene)-5-(ethyl- sulfonylamino)-2-indolinone;
- (D): (Z)-3-(1-(4-(piperidin-1-yl-methyl)-phenylamino)-1-phenyl-methylene)-5- (phenylsulfonylamino)-2- indolinone;
- (E): (Z)-3-(1-(4-(piperidin-1-yl-methyl)-phenylamino)-1-phenyl-methylene)-5-(4- amino-phenylsulfonylamino)-2-indolinone;
- (F): (Z)-3-(1-(4-(pyrrolidin-1-yl-methyl)-phenylamino)-1-phenyl-methylene)-5- (ethylsulfonylamino)-2-indolinone;
- (G): (Z)-3-(1-(4-(4-(3-aminopropyl-piperidin-1-yl-methyl)-phenylamino)-1-phenyl- methylene)-5-(ethylsulfonylamino)-2-indolinone;
- (H): (Z)-3-(1-(4-(N-(piperidin-1-yl-methylcarbonyl)-N-methyl-amino)-phenylamino)- 1-phenyl-methylene)-5-(phenylsulfonylamino)-2-indolinone;
- (I): (Z)-3-(1-(4-(N-(2-dimethylamino-ethyl)-N-methylsulfonyl-amino)-phenylamino)- 1-phenyl-methylene)-5-(N-methyl-N-phenylsulfonyl-amino)-2-indolinone;
- (J): (Z)-3-(1-(4-(N-methyl-N-(piperidin-1-yl-methylcarbonyl)-amino)-phenylamino)- 1-phenyl-methylene)-5-(N-methyl-N-phenylsulfonyl-amino)-2-indolinone;
- (K): (Z)-3-(1-(2-benzimidazolyl-amino)-1-phenyl-methylene)-5-amido-2-indolinone;
- (L): (Z)-3-(1-(4-(N-methyl-propionylamino)-phenylamino)-1-phenyl-methylene)-5- amido-2-indolinone;
- (M): (Z)-3-(1-(4-(N-(2-dimethylamino-ethyl)-N-methylsulfonyl-amino)-phenylamino)- 1-phenyl-methylene)-2-indolinone;
- (N): (Z)-3-(1-(4-(N-(3-dimethylaminopropyl)-N-propionyl-amino)-phenylamino)-1- phenyl-methylene)-2-indolinone;
- (O): (Z)-3-(1-(4-(dimethy)amhomethyl)-phenylarnino)-1-phenyl-methylene)-5- (butylcarbamoyl)-2-indolinone;
- (P): (Z)-3-(1-(4-(dimethylaminomethyl)-phenylamino)-1-phenyl-methylen)-5- (naphth-1-yl-methyl-carbamoyl)-2-indolinone;
- (Q): (Z)-3-(1-(4-(dimethylaminomethyl)-phenylamino)-1-phenyl-mothylene)-5-(N- butyl-N-phenyl-carbamoyl)-2-indolinone;
- (R): (Z)-3-(1-(4-(dimethylaminomethyl)-phenylamino)-1-phenyl-methylen)-5-(hexyl- carbamoyl)-2-indolinone;
- (S): (Z)-3-(1-(4-(dimethylaminomethyl)-phenylamino)-1-phenyl-methylen)-5- (cyclohexylmethyl-carbamoyl)-2-indolinone;
- (T): (Z)-3-(1-(4-(N-methylsulfonyl-N-(2-dimethylamino-ethyl)-amino)-phenylamino)- 1-phenyl-methylen)-5-(cyclohexylmethyl-carbamoyl)-2-indolinone;
- (U): (Z)-3-(1-(4-(buty)aminomethyl)-phenylamino)-1-phenyl-methylen)-5-(cyclo- hexylmethyl-carbamoyl)-2-indolinone;
- (V): (Z)-3-(1-(4-(pyrrolidin-1-yl-methyl)-phenylamino)-1-phenyl-methylen)-5-(cyclo- hexylmethyl-carbamoyl)-2-indolinone;
- (W): (Z)-3-(1-(4-(diethylaminomethyl)-phenylamino)-1-phenyl-methylen)-5-(cydo- hexylmethyl-carbamoyl)-2-indolinone;
- (X): (Z)-3-(1-(4-(diethylaminomethyl)-phenylamino)-1-phenyl-methylen)-5-(N-(3- chlorobenzyl)-carbamoyl)-2-indolinone;
- (Y): (Z)-3-(1-(4-(diethanolaminomethyl)-phenylamino)-1-phenyl-methylen)-5-(butyl- carbamoyl)-2-indolinone;
- (Z): (Z)-3-(1-(4-(dimethylaminomethyl)-phenylamino)-1-phenyl-methylen)-5-(N-(3- chlorobenzyl)-carbamoyl)-2-indolinone;
- (AA): (Z)-3-(1-(4-(N-acetyl-N-(2-dimethylamino-ethyl)-amino)-phenylamino)-1- phenyl-methylen)-5-(N-(3-chlorobenzyl)-carbamoyl)-2-indolinone;
- (AB): (Z)-3-(1-(4-(butylaminomethyl)-phenylamino)-1-phenyl-methylen)-5-(N-(3- chlorobenzyl)-carbamoyl)-2-indolinone;
- (AC): (Z)-3-(1-(4-(piperidin-1-yl-methyl)-phenylamino)-1-phenyl-methylene)-5-(N- methyl-N-phenyl-aminosulfonyl)-2-indolinone;
- (AD): (Z)-3-(1-(4-(piperidin-1-yl-methyl)-phenylamino)-1-phenyl-methylene)-5-(N- butyl-N-methyl-aminosulfonyl)-2-indolinone ;
- (AE): (Z)-3-(1-(4-(dimethylaminomethyl)-phenylamino)-1-phenyl-methylene)-6- methoxycarbonyl-2-indolinone;
- (AF): (Z)-3-(1-(4-(N-(3-dimethylamino-propyl)-N-acetyl-amino)-phenylamino)-1- phenyl-methylene)-6-methoxycarbonyl-2-indolinone;
- (AG): (Z)-3-(1-(4-(ethylaminomethyl)-phenylamino)-1-phenyl-methylene)-6- methoxycarbonyl-2-indolinone;
- (AH): (Z)-3-(1-(4-(1-methyl-imidazol-2-yl)-phenylamino)-1-phenyl-methylene)-6- methoxycarbonyl-2-indolinone;
- (AI): (Z)-3-(1-(4-(N-(dimethylaminomethylcarbonyl)-N-methyl-amino)-phenylamino)- 1-phenyl-methylene)-6-methoxycarbonyl-2-indolinone;
- (AJ): (Z)-3-(1-(4-(methylaminomethyl)-anilino)-1-phenyl-methylene)-6-methoxycar- bonyl-2-indolinone;
- (AK): (Z)-3-(1-(4-(N-((4-methy)-piperazin-1-yl)-methy)carbonyl)-N-methyl-amino)- phenylamino)-1-phenyl-methylene]-6-methoxycarbonyl-2-indolinone; and
- (AL): 4-(4-bromo-2-fluoroanilino)-6-methoxy-7-(1-methylpiperidin-4-ylmethoxy)- quinazoline

the tautomers, the stereoisomers and the physiologically acceptable salts thereof,

are effective inhibitors of Lck and therefore are especially suitable and effective in the treatment of immunologic diseases or pathological conditions involving an immunologic component.

Compounds (A) to (J) are described in WO 02/36564, compounds (K) to (L) are described in WO 99/52869, compounds (M) to (N) are described in WO 00/18734, compounds (O) to (AB) are described in WO 00/73297, compounds (AC) to (AD) are described in WO 01/27080, compounds (AE) to (AK) are described in WO 01/27081, compound (AL) is described in WO 01/32651.

Viewed from a first aspect the present invention provides a method for treating immunologic diseases or pathological conditions involving an immunologic component comprising administering to a patient in need of such treatment an effective amount of a pharmaceutical composition comprising a compound selected from compounds (A) to (AL), the tautomers, the stereoisomers and the physiologically acceptable salts thereof, optionally in combination with one or more other drugs selected from NSAIDs, steroids, DMARDs, immunsuppressives, biologic response modifiers and antinfectives.

The expression "patient" is meant to comprise the human and the non-human mammal patient.

The indication "immunologic diseases or pathological conditions involving an immunologic component" should be understood in a non-limiting manner to comprise

autoimmune diseases, for instance inflammatory diseases having an autoimmune component such as inflammatory diseases selected from

inflammatory bowel disease (e.g. colitis ulcerosa and Morbus Crohn), rheumatoid arthritis, glomerulonephritis and lung fibrosis,
furthermore, psoriasis, psoriasis arthritis, hypersensitivity reactions of the skin, atherosclerosis, restenosis, asthma, multiple sclerosis and type 1 diabetes,
and indications which need immunosuppressant therapy, for instance prevention or therapy of tissue or organ transplant rejection, e.g. acute or chronic graft-versus-host disease, allograft or xenograft rejection etc. The transplanted organ may be kidney, heart, liver, lung, bone marrow, peripheral blood stem cells, pancreas or islet cells thereof, cornea, small bowel, skin, or heart valve.

Preferred indications which may be treated by the method according to the invention are
rheumatoid arthritis,
inflammatory bowel disease such as colitis ulcerosa and Morbus Crohn,
psoriasis, psoriasis arthritis,
prevention or therapy of tissue or organ transplant rejection, acute or chronic graft-versus-host disease, allograft or xenograft rejection,
allergic asthma, multiple sclerosis and type 1 diabetes.

A further subgroup of indications which may be treated by the method according to the invention and deserves special mention comprises morbus crohn, lung fibrosis, psoriasis arthritis, hypersensitivity reactions of the skin, graft-versus-host disease (acute and chronic), asthma, multiple sclerosis and type 1 diabetes.

A preferred embodiment of the method according to the invention comprises administration of a compound selected from compounds
(A), (B), (C), (D), (F), (G), (P), (T), (V), (X), (Z), (AA), (AE), (AI), (AK) and (AL),
the tautomers, the stereoisomers and the physiologically acceptable salts thereof, optionally in combination with one or more other drugs selected from NSAIDs, steroids, DMARDs, immunsuppressives, biologic response modifiers and antinfectives.

Another preferred embodiment of the method according to the invention comprises administration of a compound selected from the following combined inhibitors of VEGFR-2 and Lck
(M), (N), (O), (S), (T), (U), (V), (W), (X), (Y), (Z), (AA), (AB), (AE), (AF), (AG), (AH), (AI), (AJ), (AK) and (AL),
the tautomers, the stereoisomers and the physiologically acceptable salts thereof, optionally in combination with one or more other drugs selected from NSAIDs, steroids, DMARDs, immunsuppressives, biologic response modifiers and antinfectives. Since VEGF also plays an important pathogenetical role in chronic inflammatory bowel diseases such as colitis ulcerosa and morbus crohn as well as in rheumatoid arthritis, psoriasis and psoriasis arthritis these combined inhibitors of VEGFR-2 and Lck are of special advantage in these most preferred indications.

A further preferred embodiment of the method according to the invention comprises administration of a compound selected from compounds
(AK), (AI) and (AL),
the tautomers, the stereoisomers and the physiologically acceptable salts thereof,
especially preferred is administration of compound (AK).

In the method of treatment according to the invention compounds (A) to (AL) can be administered orally, parenterally, rectally or, with respect to indications involving treatment of the skin such as psoriasis, psoriasis arthritis or hypersensitivity reactions of the skin, also in topical formulations. Oral administration is preferred.

In oral, rectal or topical administration the compounds may be given, if required in divided doses, in a daily dosage of 0.1 to 20 mg/kg body weight, preferably 0.5 to 20 mg/kg body weight, most preferred 1 to 10 mg/kg body weight.

Parenterally the compounds may be administered in lower doses, for instance in a total daily dosage of 0.01 to 5 mg/kg body weight, preferably 0.05 to 2 mg/kg body weight, most preferred 0.1 to 1 mg/kg body weight.

For administration the compounds may be formulated with one or more conventional inert carriers and/or diluents as known in the art, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethylene glycol, propylene glycol, stearyl alcohol, carboxymethylcellulose or fatty substances such as hard fat or suitable mixtures thereof in conventional galenic preparations such as plain or coated tablets, lozenges, hard or soft capsules, dispersible powders or granules, syrups or elixirs, injectable solutions, ampoules, aqueous or oily suspensions, emulsions, solutions, sprays, creams, ointments, gels, or suppositories. Suitable galenic formulations are disclosed in the documents cited hereinbefore.

Furthermore, in the method according to the invention a compound selected from compounds (A) to (AL) may be administered in combination, simultaneously or sequentially, with one or more other drugs selected from NSAIDs, steroids, DMARDs, immunsuppressives, biologic response modifiers, antinfectives and, in case of lung indications, also with bronchodilators.

In particular a compound selected from compounds (A) to (AL) could be used in combination with immunosuppressives in the prevention or treatment of the acute rejection of transplanted organs,

in combination with NSAIDs, steroids, immunosupressives, DMARDs, biologic response modifiers (e.g. Anti-TNF), and antiinfectives for the treatment of inflammatory bowel disease (e.g. colitis ulcerosa and morbus crohn), rheumatoid arthritis and psoriasis, whereby the NSAID-dose can be significantly reduced compared to the normally needed dose to produce a therapeutic effect thus reducing the risk of adverse side-effects from the NSAID such as gastrointestinal effects,

in combination with biologic response modifiers ( e.g. leukotriene antagonists) and bronchodilators for the treatment of asthma,

Suitable NSAIDs for combination treatment are meant to include all COX (cyclooxygenase) inhibitors, e.g.
non-selective COX-inhibitors such as acetylsalicyclic acid, mesalazin, ibuprofen, naproxen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen,

indomethacin, sulindac, tolmetin, zomepirac, nabumetone, diclofenac, fenclofenac, alclofenac, bromfenac, ibufenac, aceclofenac, acemetacin, fentiazac, clidanac, etodolac, oxpinac,
mefenamic acid, meclofenamic acid, flufenamic acid, nifluminic acid, tolfenamic acid, diflunisal, flufenisal, piroxicam, tenoxicam, lornoxicam and nimesulide and the pharmaceutically acceptable salts thereof,
as well as selective COX 2-inhibitors such as meloxicam, celecoxib and rofecoxib and the pharmaceutically acceptable salts thereof.

Suitable steroids for combination treatment are meant to include in a non-limiting manner prednisone, prednisolone, methylprednisolone, dexamethasone, budenoside, fluocortolone and triamcinolone.

Suitable DMARDs for combination treatment are meant to include in a non-limiting manner sulfasalazine, olsalazine, chloroquin, gold derivatives (Auranofin), D-penicillamine and cytostatics such as methotrexate and cyclophosphamide.

Suitable immunsuppressives for combination treatment are meant to include in a non-limiting manner cyclosporin A and derivatives thereof, mycophenolatemofetil, FK 506, OKT-3, ATG, 15-desoxyspergualin, mizoribine, misoprostol, rapamycin, reflunomide, azathioprine and NF-Kappa B-inhibitors.

Suitable biologic response modifiers for combination treatment are meant to include in a non-limiting manner interferon beta, anti-TNF-alpha (Etanercept), IL-10, oral and parenteral tolerance induction strategies (orally e.g. with genetically modified enteric bacteria), leukotrien-antagonists, anti-CD3 or anti-CD25.

Suitable antinfectives for combination treatment are meant to include in a non-limiting manner metronidazol and chinolone for treatment of chronic inflammatory bowel diseases.

Suitable bronchodilators for combination treatment are meant to include in a non-limiting manner those disclosed under "broncholytics/antiasthmatics" in Rote Liste® 2002, Editio Cantor Verlag Aulendorf, Germany, being herewith incorporated by reference, for instance ipratropiumbromide, oxytropiumbromide, tiotropiumbromide, epinephrinehydrochloride, salbutamole, terbutalinsulfate, fenoterolhydrobromide, salmeterole, formoterole, cromiclinic acid, theophylline derivatives etc.

In such combinations each active: ingredient can be administered either in accordance with its usual dosage range or a dose below its usual dosage range. The dosage for the combined NSAIDs, steroids, DMARDs, immunsuppressives, biologic response modifiers and antinfectives is appropriately 1/50 of the lowest dose normally recommended up to 1/1 of the normally recommended dosage, preferably 1/20 to 1/2 and more preferably 1/10 to 1/5. The normally recommended dose for the combined drug should be understood to be the dose disclosed for example in Rote Liste^{®} 2002, Editio Cantor Verlag Aulendorf, Germany, or in Physician's Desk Reference.

It can be expected that combination treatment comprising administration of Lck inhibitors together with a second drug selected from those mentioned hereinbefore may provide synergistic efficacy thus allowing significant dose reduction compared to the normally needed dose to produce a therapeutic effect. This would be especially beneficial with regard to medications having a high risk of adverse side-effects as is the case with non-selective COX inhibitors, cyclosporin A or DMARDs.

Viewed from a second aspect the present invention also relates to pharmaceutical composition comprising
(a) a compound selected from compounds (A) to (AL), the tautomers, the stereoisomers and the physiologically acceptable salts thereof,
(b) and one or more other drugs selected from NSAIDs, steroids, DMARDs, immunsuppressives, biologic response modifiers and antinfectives,
optionally together with pharmaceutically acceptable diluents and/or carriers, as a combined preparation or a kit of parts containing components (a) and (b) in separate containments for simultaneous, separate or sequential use in treatment of immunologic diseases or pathological conditions involving an immunologic component.

Viewed from a third aspect the present invention provides the use of a compound selected from compounds (A) to (AL), the tautomers, the stereoisomers and the physiologically acceptable salts thereof, optionally in combination with one or more other drugs selected from NSAIDs, steroids, DMARDs, immunsuppressives, biologic response modifiers and antinfectives, for the manufacture of a pharmaceutical composition for the treatment of a patient suffering from immunologic diseases or pathological conditions involving an immunologic component.

Preferred embodiments of either the composition aspect or the use aspect of the invention with respect to the combined VEGFR-2/Lck component correspond to those mentioned hereinbefore for the method of treatment aspect.

### Example 1: Non-radioactive kinase assay (lck)

### Methodology

The lck enzyme comprises the entire lck molecule except the first nine amino acids which are replaced by an His-tag for purification purposes. The enzyme is affinity purified.

The assay mix is assembled in a well of a 96-well round bottom microtiter plate and contains 10 µl PBS (either as such or with an inhibitor dissolved at an appropriate concentration), 20 µl substrate solution (200 mM Hepes, pH=7,4; 50 mM MgAc₂; 1 mM Na₃VO₄; 250 µg/ml poly-Glu-Tyr (Sigma P0275); 200 ng/ml biotinylated peptide (biot-Ala-Glu-Glu-Gtu-Ile-Tyr-Gly-Glu-Phe-Glu-Ala-Lys-Lys-Lys-Lys) and 20 µl of 2,5 ng/µl enzyme (diluted from affinity purified stock with enzyme dilution buffer, EDB: 20 mM Hepes, pH=7,4, 130 mM NaCl, 0,05% Triton X-100).

The reaction is started by the addition of 50 µl 500 µM ATP (in 10 mM MgAc₂) and is performed at room temperature. After 30 minutes 50 µl stop solution (20 mM Hepes, pH=7,4;250 mM EDTA) are added and 100 µl of this solution transfered to the well of a streptavidin coated microtiter plate (SA-MTP, Boehringer Mannheim, #1664-760). The solution is incubated for one hour at room temparature and the supernatant discarded. The well is washed twice with 300 µl PBS.

The streptavidin bound biotinylated peptide is incubated for 1 hour at room temperature with 100 µl Eu³⁺-labelled anti-phosphotyrosine antibody solution (0,3 mg/ml DELFIA-Eu-labelled PT66 (Wallac, AD0041); 50 mM Tris, pH=7,8; 0,05% Tween 20; 0,5% (w7v) BSA (Serva, diagnostic grade) under gentle agitation. The well is washed three times with 1 x Delfia wash buffer (Wallac, 1244-114, 25x concentrate, diluted with water) and finally 100 µl Delfia enhancement solution (Wallac, 1244-105) are added.

Time resolved fluorescense is measured in a Wallac Victor2 1420 Multilabel Counter, excitation is at 340 nm, emission is measured at 615 nm (delay time 400 µsec, window time 1000 µsec).

### Results

In two independent experiments the IC₅₀s of compounds (A) to (AL) on the kinase have been determined. The data (mean values) obtained with three representative compounds are summarised in the following table:

| compound | Lck; IC₅₀ *[nM]* |
|---|---|
| (AK) | 16 |
| (AI) | 36 |
| (AL) | 58 |

Compounds (A) to (AH) and (AJ) inhibit the lck kinase function with an IC₅₀< 1 µM.

## Claims

1. A method for treating immunologic diseases or pathological conditions involving an immunologic component comprising administering to a patient in need of such treatment an effective amount of a pharmaceutical composition comprising a compound selected from
(A) (Z)-3-(1-(4-(piperidin-1-yl-methyl)-phenylamino)-1-phenyl-methylene)-5- (methylsulfonylamino)-2-indolinone;
(B) (Z)-3-(1-(4-(pipefidin-1-yl-methyl)-phenylamino)-1-phenyl-mothylene)-5-(ethyl- sulfonylamino)-2-indolinone;
(C) (Z)-3-(1-(4-(dimethylaminomethyl)-phenylamino)-1-phenyl-methylene)-5-(ethyl- sulfonylamino)-2-indolinone;
(D) (Z)-3-(1-(4-(piperidin-1-yl-methyl)-phenylamino)-1-phenyl-methylene)-5- (phenylsulfonylamino)-2-indolinone;
(E) (Z)-3-(1-(4-(piperidin-1-yl-methyl)-phenylamino)-1-phenyl-methylene)-5-(4- amino-phenylsulfonylamino)-2-indolinone;
(F) (Z)-3-(1-(4-(pyrrolidin-1-yl-methyl)-phenylamino)-1-phenyl-methylene)-5- (ethylsulfonylamino)-2-indolinone;
(G) (Z)-3-(1-(4-(4-(3-aminopropyl-piperidin-1-yl-methyl)-phenylamino)-1-phenyl- methylene)-5-(ethylsulfonylamino)-2-indolinone;
(H) (Z)-3-(1-(4-(N-(piperidin-1-yl-methylcarbonyl)-N-methyl-amino)-phenylamino)- 1-phenyl-methylene)-5-(phenylsulfonyfamino)-2-indolinone;
(I) (Z)-3-(1-(4-(N-(2-dimethylamino-ethyl)-N-methylsulfonyl-amino)-phenylamino)- 1-phenyl-methylene)-5-(N-methyl-N-phenylsulfonyl-amino)-2-indolinone;
(J) (Z)-3-(1-(4-(N-methyl-N-(piperidin-1-yl-methylcarbonyl)-amino)-phenylamino)- 1-phenyl-methylene)-5-(N-methyl-N-phenylsulfonyl-amino)-2-indolinone;
(K) (Z)-3-(1-(2-benzimidazolyl-amino)-1-phenyl-methylene)-5-amido-2-indolinone;
(L) (Z)-3-(1-(4-(N-methy)-propionylamino)-phenylamino)-1-phenyl-methylene)-5- amido-2-indolinone;
(M) (Z)-3-(1-(4-(N-(2-dimethylamino-ethyl)-N-methylsulfonyl-amino)-phenylamino)- 1-phenyl-methylene)-2-indolinone;
(N) (Z)-3-(1-(4-(N-(3-dimethylaminopropyl)-N-propionyl-amino)-phenylamino)-1- phenyl-methylene)-2-indolinone;
(O) (Z)-3-(1-(4-(dimethylaminomethyl)-phenylamino)-1-phenyl-methylene)-5- (butylcarbamoyl)-2-indolinone;
(P) (Z)-3-(1-(4-(dimethylaminomethyl)-phenylamino)-1-phenyl-methylen)-5- (naphth-1-yl-methyl-carbamoyl)-2-indolinone;
(Q) (Z)-3-(1-(4-(dimethylaminomethyl)-phenylamino)-1-phenyl-methylene)-5-(N- butyl-N-phenyl-carbamoyl)-2-indolinone;
(R) (Z)-3-(1-(4-(dimethylaminomethyl)-phenylamino)-1-phenyl-methylen)-5-(hexyl- carbamoyl)-2-indolinone;
(S) (Z)-3-(1-(4-(dimethylaminomethyl)-phenylamino)-1-phenyl-methylen)-5- (cyclohexylmethyl-carbamoyl)-2-indolinone;
(T) (Z)-3-(1-(4-(N-methylsulfonyl-N-(2-dimethylamino-ethyl)-amino)-phenylamino)- 1-phenyl-methylen)-5-(cyclohexylmethyl-carbamoyl)-2-indolinone;
(U) (Z)-3-(1-(4-(butylaminomethyl)-phenylamino)-1-phenyl-methylen)-5-(cyclo- hexylmethyl-carbamoyl)-2-indolinone;
(V) (Z)-3-(1-(4-(pyrrolidin-1-y)-methyl)-phenylamino)-1-phenyl-methylen)-5-(cyclo- hexylmethyl-carbamoyl)-2-indolinone;
(W) (Z)-3-(1-(4-(diethylaminomethyl)-phenylamino)-1-phenyl-methylen)-5-(cyclo- hexylmethyl-carbamoyl)-2-indolinone;
(X) (Z)-3-(1-(4-(diethylaminomethyl)-phenylamino)-1-phenyl-methylen)-5-(N-(3-chlorobenzyl)-carbamoyl)-2-indolinone;
(Y) (Z)-3-(1-(4-(diethanolaminomethyl)-phenylamino)-1-phenyl-methylen)-5-(butyl- carbamoyl)-2-indolinone;
(Z) (Z)-3-(1-(4-(dimethylaminomethyl)-phenylamino)-1-phenyl-methylen)-5-(N-(3- chlorobenzyl)-carbamoyl)-2-indolinone;
(AA) (Z)-3-(1-(4-(N-acetyl-N-(2-dimethylamino-ethyl)-amino)-phenylamino)-1- phenyl-methylen)-5-(N-(3-chlorobenzyl)-carbamoyl)-2-indolinone;
(AB) (Z)-3-(1-(4-(butylaminomethyl)-phenylamino)-1-phenyl-methylen)-5-(N-(3- chlorobenzyl)-carbamoyl)-2-indolinone;
(AC) (Z)-3-(1-(4-(piperidin-1-yl-methyl)-phenylamino)-1-phenyl-methylene)-5-(N- methyl-N-phenyl-aminosulfonyl)-2-indolinone;
(AD) (Z)-3-(1-(4-(piperidin-1-yl-methyl)-phenylamino)-1-phenyl-methylene)-5-(N- butyl-N-methyl-aminosulfonyl)-2-indolinone ;
(AE) (Z)-3-(1-(4-(dimethylaminomethyl)-phenylamino)-1-phenyl-methylene)-6- methoxycarbonyl-2-indolinone;
(AF) (Z)-3-(1-(4-(N-(3-dimethylamino-propyl)-N-acetyl-amino)-phenylamino)-1- phenyl-methylene)-6-methoxycarbonyl-2-indolinone;
(AG) (Z)-3-(1-(4-(ethylaminomethyl)-phenylamino)-1-phenyl-methylene)-6- methoxycarbonyl-2-indolinone;
(AH) (Z)-3-(1-(4-(1-methyl-imidazol-2-yl)-phenylamino)-1-phenyl-methylene)-6- methoxycarbonyl-2-indolinone;
(AI) (Z)-3-(1-(4-(N-(dimethylaminomethylcarbonyl)-N-methyl-amino)-phenylamino)- 1-phenyl-methylene)-6-methoxycarbonyl-2-indolinone;
(AJ) (Z)-3-(1-(4-(methylaminomethyl)-anilino)-1-phenyl-methylene)-6-methoxycar- bonyl-2-indolinone;
(AK) (Z)-3-(1-(4-(N-((4-methyl-piperazin-1-yl)-methylcarbonyl)-N-mothyl-amino)- phenylamino)-1-phenyl-methylene]-6-methoxycarbonyl-2-indolinone; and
(AL) 4-(4-bromo-2-fluoroanilino)-6-methoxy-7-(1-methylpiperidin-4-ylmethoxy)- quinazoline
the tautomers, the stereoisomers and the physiologically acceptable salts thereof, optionally in combination with one or more other drugs selected from nonsteroidal anti-inflammatory drugs (NSAIDs), steroids, disease-modifying antirheumatic drugs (DMARDs), immunsuppressives, biologic response modifiers and antinfectives.

2. The method of claim 1, wherein the immunologic disease or pathological condition involving an immunologic component is selected from
autoimmune diseases, for instance inflammatory diseases having an autoimmune component such as inflammatory diseases selected from
inflammatory bowel disease (e.g. colitis ulcerosa and Morbus Crohn), rheumatoid arthritis, glomerulonephritisand lung fibrosis,
furthermore, psoriasis, psoriasis arthritis, hypersensitivity reactions of the skin, atherosclerosis, restenosis, asthma, multiple sclerosis and type 1 diabetes,
and indications which need immunosuppressant therapy, for instance prevention or therapy of tissue or organ transplant rejection.

3. The method of claim 2, wherein the immunologic disease or pathological condition involving an immunologic component is selected from
rheumatoid arthritis,
inflammatory bowel disease such as colitis ulcerosa and Morbus Crohn, psoriasis, psoriasis arthritis,
prevention or therapy of tissue or organ transplant rejection, acute or chronic graft-versus-host disease, allograft or xenograft rejection,
allergic asthma, multiple sclerosis and type 1 diabetes.

4. The method of claim 2, wherein the immunologic disease or pathological condition involving an immunologic component is selected from morbus crohn, lung fibrosis, psoriasis arthritis, hypersensitivity reactions of the skin, graft-versus-host disease (acute and chronic), asthma, multiple sclerosis and type 1 diabetes.

5. The method of claim 2, wherein the immunologic disease or pathological condition involving an immunologic component is selected from chronic inflammatory bowel diseases, such as colitis ulcerosa and morbus crohn, from rheumatoid arthritis, psoriasis and psoriasis arthritis.

6. The method according to any of claims 1 to 5, which method comprises administration of a compound selected from
(A), (B), (C), (D), (F), (G), (P), (T), (V), (X), (Z), (AA), (AE), (AI), (AK) and (AL),
the tautomers, the stereoisomers and the physiologically acceptable salts thereof, optionally in combination with one or more other drugs selected from NSAIDs, steroids, DMARDs, immunsuppressives, biologic response modifiers and antinfectives.

7. The method of claim 5, which method comprises administration of a compound selected from
(M), (N), (O), (S), (T), (U), (V), (W), (X), (Y), (Z), (AA), (AB), (AE), (AF), (AG), (AH), (Al), (AJ), (AK) and (AL),
the tautomers, the stereoisomers and the physiologically acceptable salts thereof, optionally in combination with one or more other drugs selected from NSAIDs, steroids, DMARDs, immunsuppressives, biologic response modifiers and antinfectives.

8. The method according to any of claims 1 to 5, which method comprises administration of a compound selected from (AK), (Al) and (AL) mentioned in claim 1, the tautomers, the stereoisomers and the physiologically acceptable salts thereof.

9. The method according to any of claims 1 to 8, wherein the compounds are administered orally, parenterally, rectally or, with respect to indications involving treatment of the skin such as psoriasis, psoriasis arthritis or hypersensitivity reactions of the skin, also topically.

10. The method of claim 9, wherein in oral, rectal or topical administration the compounds are given in a daily dosage of 0.1 to 20 mg/kg body weight or in parenteral administration in a total daily dosage of 0.01 to 5 mg/kg body weight.

11. A pharmaceutical composition comprising
(a) a compound selected from compounds (A) to (AL) of claim 1, the tautomers, the stereoisomers and the physiologically acceptable salts thereof,
(b) and one or more other drugs selected from NSAIDs, steroids, DMARDs, immunsuppressives, biologic response modifiers and antinfectives,
optionally together with pharmaceutically acceptable diluents and/or carriers, as a combined preparation or a kit of parts containing components (a) and (b) in separate containments for simultaneous, separate or sequential use in treatment of immunologic diseases or pathological conditions involving an immunologic component.

12. The composition of claim 11, wherein the NSAID is a non-selective COX-inhibitor or a COX-2 selective inhibitor.

13. The composition of claim 11, wherein the NSAID is selected from acetylsalicyclic acid, mesalazin, ibuprofen, naproxen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen,
indomethacin, sulindac, tolmetin, zomepirac, nabumetone, diclofenac, fenclofenac, alclofenac, bromfenac, ibufenac, aceclofenac, acemetacin, fentiazac, clidanac, etodolac, oxpinac,
mefenamic acid, meclofenamic acid, flufenamic acid, nifluminic acid, tolfenamic acid, diflunisal, flufenisal, piroxicam, tenoxicam, lornoxicam, nimesulide,
meloxicam, celecoxib and rofecoxib,
and the pharmaceutically acceptable salts thereof.

14. The composition of claim 11, wherein the steroid is selected from
prednisone, prednisolone, methylprednisolone, dexamethasone, budenoside, fluocortolone and triamcinolone.

15. The composition of claim 11, wherein the DMARD is selected from sulfasalazine, olsalazine, chloroquin, gold derivatives (Auranofin), D-penicillamine and cytostatics such as methotrexate and cyclophosphamide.

16. The composition of claim 11, wherein the immunsuppressive is selected from cyclosporin A and derivatives thereof, mycophenolatemofetil, FK 506, OKT-3, ATG, 15-desoxyspergualin, mizoribine, misoprostol, rapamycin, reflunomide, azathioprine and NF-Kappa B-inhibitors.

17. The composition of claim 11, wherein the biologic response modifier is selected from interferon beta, anti-TNF-alpha (Etanercept), IL-10, oral and parenteral tolerance induction strategies, leukotrien-antagonists, anti-CD3 and anti-CD25.

18. The composition of claim 11, wherein the antinfective is selected from metronidazol and chinolone.

19. The composition of claim 11, wherein the bronchodilator is selected from ipratropiumbromide, oxytropiumbromide, tiotropiumbromide, epinephrinehydrochloride, salbutamole, terbutalinsulfate, fenoterolhydrobromide, salmeterole, formoterole, cromiclinic acid, theophylline derivatives.

20. Use of a compound selected from effective compounds (A) to (AL) mentioned in claim 1, the tautomers, the stereoisomers and the physiologically acceptable salts thereof, optionally, in combination with one or more other drugs selected from NSAIDs, steroids, DMARDs, immunsuppressives, biologic response modifiers and antinfectives, for the manufacture of a pharmaceutical composition for the treatment of a patient suffering from immunologic diseases or pathological conditions involving an immunologic component.

21. The use of claim 20, wherein the immunologic disease or pathological condition involving an immunologic component is selected from
autoimmune diseases, for instance inflammatory diseases having an autoimmune component such as inflammatory diseases selected from
inflammatory bowel disease (e.g. colitis ulcerosa and Morbus Crohn), rheumatoid arthritis, glomerulonephritisand lung fibrosis,
furthermore, psoriasis, psoriasis arthritis, hypersensitivity reactions of the skin, atherosclerosis, restenosis, asthma, multiple sclerosis and type 1 diabetes,
and indications which need immunosuppressant therapy, for instance prevention or therapy of tissue or organ transplant rejection.

22. The use of claim 20, wherein the immunologic disease or pathological condition involving an immunologic component is selected from
rheumatoid arthritis,
inflammatory bowel disease such as colitis ulcerosa and Morbus Crohn, psoriasis, psoriasis arthritis,
prevention or therapy of tissue or organ transplant rejection, acute or chronic graft-versus-host disease, allograft or xenograft rejection,
allergic asthma, multiple sclerosis and type 1 diabetes.

23. The use of claim 20, wherein the immunologic disease or pathological condition involving an immunologic component is selected from morbus crohn, lung fibrosis, psoriasis arthritis, hypersensitivity reactions of the skin, graft-versus-host disease (acute and chronic), asthma, multiple sclerosis and type 1 diabetes.

24. The use of claim 20, wherein the immunologic disease or pathological condition involving an immunologic component is selected from chronic inflammatory bowel diseases, such as colitis ulcerosa and morbus crohn, from rheumatoid arthritis, psoriasis and psoriasis arthritis.

25. The use of claim 20, wherein the compound is selected from the following combined inhibitors of VEGFR-2 und Lck
(M), (N), (O), (S), (T), (U), (V), (W), (X), (Y), (Z), (AA), (AB), (AE), (AF), (AG), (AH), (AI), (AJ), (AK) and(AL) mentioned in claim 1,
the tautomers, the stereoisomers and the physiologically acceptable salts thereof,
and wherein the indication to be treated is selected from chronic inflammatory bowel diseases, such as colitis ulcerosa and morbus crohn, from rheumatoid arthritis, psoriasis and psoriasis arthritis.

26. The use of a compound selected from
(A), (B), (C), (D), (F), (G), (P), (T), (V), (X), (Z), (AA), (AE), (AI) and (AL) mentioned in claim 1,
the tautomers, the stereoisomers and the physiologically acceptable salts thereof, optionally in combination with one or more other drugs selected from NSAIDs, steroids, DMARDs, immunsuppressives, biologic response modifiers and antinfectives,
according to any of claims 20 to 24.

27. The use of a compound selected from (AK), (AI) and (AL) mentioned in claim 1, the tautomers, the stereoisomers and the physiologically acceptable salts thereof, according to any of claims 20 to 24.

28. The use according to any of claims 20 to 25 for the manufacture of a pharmaceutical composition adapted for oral, parenteral, rectal or topical administration.

29. The use of claim 26, wherein the pharmaceutical composition for oral, rectal or topical administration is adapted for administration of a daily dosage of the effective compound of 0.1 to 20 mg/kg body weight or, for parenteral administration, is adapted for a total daily dosage of 0.01 to 5 mg/kg body weight.
